# EUROPEAN PATENT APPLICATION

(11) **EP 2 668 924 A1**
(43) Date of publication of application: **04.12.2013**
(21) Application number: 12739241.3
(22) Date of filing: 13.01.2012
(51) Int. Cl.: A61B 19/00, A61F 2/02, A61L 27/00

(54) **PLATELET RICH FIBRIN-FLATTENING DEVICE**

(30) Priority: 28.01.2011 JP 2011016878
(71) Applicant: Niigata University, Niigata-shi Niigata 950-2181 (JP); YSEC Company, Limited, Kanagawa 240-0051 (JP)
(72) Inventor: KAWASE Tomoyuki, Niigata-shi Niigata 951-8514 (JP); YAMAUCHI Keijiro, Niigata-shi Niigata 953-0054 (JP); ABE Kazuyuki, Niigata-shi Niigata 953-0054 (JP)
(74) Representative: Isarpatent
(86) International application number: PCT/JP2012/050545
(87) International publication number: WO 2012/102094

(57) **Abstract**

A compression device is provided which allows anyone to easily uniformly and thinly compress fibrin-gel without damage. A fibrin-gel compression device 1 includes: a first member 10 having a handle part 11 and a pressing convex part 12; and a second member 20 having a handle part 21 and an accommodating concave part 22 forming a shape corresponding to the pressing convex part. The first and second members are partially joined or rotatably supported to each other to variably adjust a separation distance d between the pressing convex part and the accommodating concave part to be able to accommodate and compress fibrin-gel 30 therebetween. Further, it is preferable that a shape difference is provided between the convex part 12 and the concave part 22 or a stopper member is provided on at least one of the handle parts 12, 21 to provide the separation distance d of 1 mm to 2 mm when compressing the fibrin-gel 30. Further, it is preferable that a nonslip surface formed of many pinholes 25 or the like is formed at a portion of the accommodating concave part 22, the pressing convex part 12, or the concave part and the convex part.

## Description

### Technical Field

The present invention relates to a device that compresses fibrin-gel prepared from platelet rich plasma for regenerative therapy to make the fibrin-gel to have a uniform thickness.

### Background Art

Recently, platelet rich plasma (PRP) prepared from blood of a patient is applied in fields of regenerative therapy for periodontal disease and the like, implantation of artificial implant, and cosmetic surgery, and achieves good outcome from the therapy. However, the PRP is liquid and therefore inferior in operability and in the ability of continuously drawing out effects while staying in tissue in a certain region.

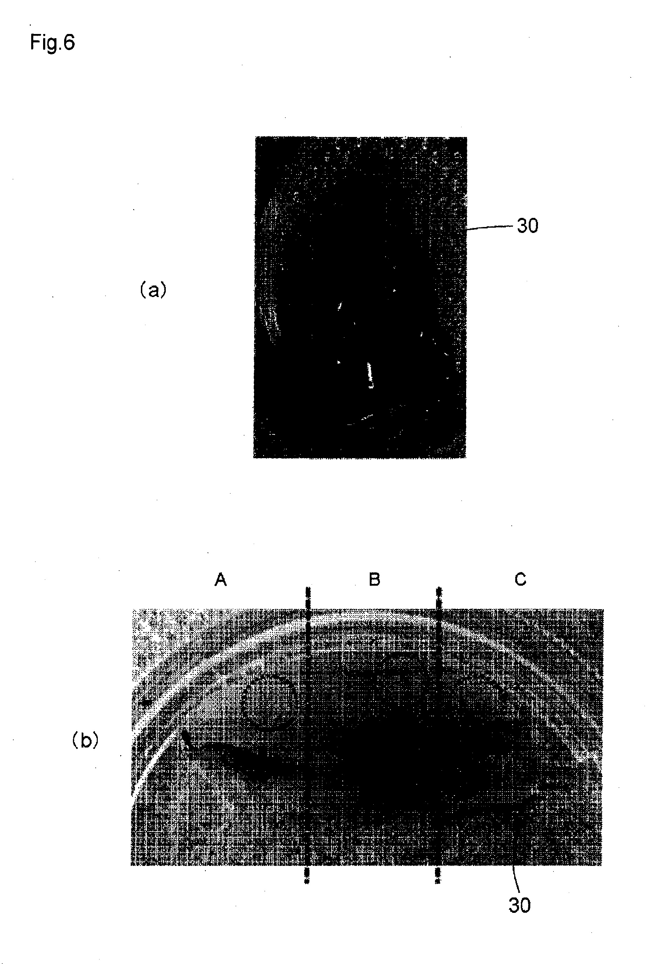

Therefore, a method of preparing gelatinous platelet rich fibrin (PRF (note that it is also referred to as " fibrin-gel" in this specification)) called a second generation PRP has been developed by Choukroun et al. so far (see Non-Patent Document 1). The fibrin-gel is a gelatinous substance prepared using the centrifugal force by a centrifuge and an inherent coagulation system of blood plasma (see FIG. 6(a)). The fibrin-gel is prepared in terms of formation state only by adjustment of the number of rotations of centrifugation and is therefore free of animal derived factors, such as bovine thrombin and free of added calcium as well as has appropriate softness and formability to be excellent also in operability.

Also in Japan, devices of converting blood plasma into a gelatinous form have been commercially available and introduced into many medical institutions and are becoming the mainstream of recent regenerative therapy for periodontal disease and the like (see Non-Patent Document 2).

Incidentally, for implanting the prepared fibrin-gel, a method of (1) slicing the fibrin-gel or (2) flattening the fibrin-gel by gauze, and the like is usually employed. However, the former method has great difficulty in slicing the gelatinous substance into a uniform thickness. On the other hand, the latter method is pointed out to have disadvantages that the gauze absorbs also growth factors and nutrients (plasma components) contained in the fibrin-gel and damages platelet adhering to the surface of the fibrin-gel.

The present inventors have been early engaged in research of the fibrin-gel, in which a method of flattening the fibrin-gel between two glass plates without using gauze was employed on a trial basis as the above-described flattening method, and when the above-described slicing method was compared with the flattening method in animal experiments, a significant wound-healing effect was found in the fibrin-gel produced by the flattening method (see Non-Patent Document 3. Note that a concrete configuration for flattening the fibrin-gel is not disclosed in Non-Patent Document 3.). However, a dedicated tool for appropriately and efficiently flattening the fibrin-gel has not been available and even the existence of the tool has been unclear.

### Prior Art Document

### Non-Patent Document

Non-Patent Document 1: Dohan DM, Choukroun J, Diss A, Dohan SL, Dohan AJ, Mouhyi J, Gogly B. Platelet-rich fibrin (PRF): a second-generation platelet concentrate. Part I: Technological concepts and evolution. Oral Surg Oral Med Oral Pathol Oral Radiol Endod, 101: e37-44, 2006.
Non-Patent Document 2: Product Pamphlet of "Blood Donation Centrifuge MEDIFUGE" (PDF), [online], Corefront Corporation website, [retrieved January 28, 2011], Internet <http://www.corefront.com/pdf/medifuge.pdf>
Non-Patent Document 3: NAKAJIMA Yu, KAWASE Tomoyuki. OKUDA Kazuhiro, YOSHIE Hiromasa, "Effect on Wound-Healing by Platelet-Rich Fibrin (PRF)" Program and Abstracts of 53th Annual Meeting in Fall, The Japanese Society of Periodontology, Annual Meeting of specified non-profit organization, The Japanese Society of Periodontology, Vol. 2010f (2010) pp.69

### Disclosure of the Invention

### Problems to Be Solved by the Invention

The present invention has been made in consideration of the above circumstances and its object is to provide a compression device allowing anyone to easily uniformly and thinly compress fibrin-gel without damage. Means for Solving the Problems

The present inventors found, after earnest study, that it is possible to provide a dedicated tool suitable for compression of fibrin-gel by using and machining into unique structures a pair of members (for example, two spoon-shaped members), one having a concave part and the other having a convex part corresponding to the concave part, and completed the present invention.

The present invention has, for example, the following configuration and characteristics.

### (Aspect 1)

A fibrin-gel compression device characterized by including:
a first member having a handle part and a pressing convex part; and
a second member having a handle part and an accommodating concave part forming a shape corresponding to the pressing convex part,
wherein the first and second members are partially joined or rotatably supported to each other to variably adjust a separation distance between the pressing convex part and the accommodating concave part to be able to accommodate and compress fibrin-gel therebetween.

### (Aspect 2)

The fibrin-gel compression device according to aspect 1,
wherein a shape difference is provided between a shape of the pressing convex part and a shape of the accommodating concave part or a stopper member is provided on at least one of the handle parts, to provide the separation distance of 1 mm to 2 mm when compressing the fibrin-gel.

### (Aspect 3)

The fibrin-gel compression device according to aspect 1 or 2,
wherein a nonslip surface is formed at a portion of the accommodating concave part, the pressing convex part, or the accommodating concave part and the pressing convex part.

### (Aspect 4)

The fibrin-gel compression device according to aspect 3,
wherein the nonslip surface is formed of many pinholes.

### (Aspect 5)

The fibrin-gel compression device according to aspect 4,
wherein the pinholes are through holes.

### (Aspect 6)

The fibrin-gel compression device according to aspect 4 or 5,
wherein the pinholes have a diameter of 0.4 mm to 1.0 mm and are provided at a hole density of 0.05 to 0.2 holes/mm².

### (Aspect 7)

The fibrin-gel compression device according to any one of aspects 1 to 6,
wherein the pressing convex part and the accommodating concave part are formed in a spoon shape.

### (Aspect 8)

The fibrin-gel compression device according to any one of aspects 1 to 7,
wherein the pressing convex part and the accommodating concave part are joined to each other at other ends of the handle parts to be superposed one above the other while variably keeping the separation distance therebetween.

### (Aspect 9)

The fibrin-gel compression device according to any one of aspects 1 to 8,
wherein the first and second members are members containing at least one of paper, ABS resin (acrylonitrile butadiene styrene copolymer synthetic resin), PE (polyethylene), PS (styrol), PVC (vinyl chloride), MA (acrylic), PET (polyethylene terephthalate), PP (polypropylene), PMP (polymethylpentene), and PC (polycarbonate), and the fibrin-gel compression device is disposable.

### (Aspect 10)

The fibrin-gel compression device according to any one of aspects 1 to 8,
wherein the first and second members are members containing at least one of stainless steel, titanium, aluminum, PP (polypropylene), PMP (polymethylpentene), and PC (polycarbonate), and the fibrin-gel compression device is able to be subjected to sterilization processing or ultrasonic cleaning.

### (Aspect 11)

The fibrin-gel compression device according to any one of aspects 1 to 10,
wherein the fibrin-gel compression device has been previously subjected to sterilization processing.

### Effect of the Invention

According to the present invention, the dedicated tool having the above configuration is used to compress the fibrin-gel without slicing it so as to increase its density, thereby improving the healing rate, namely, the healing effect of the implanted fibrin-gel (in other words, the healing ability of the implanted fibrin-gel toward its surroundings of a wound portion). It is also possible to compress the fibrin-gel into a uniform thickness without damage while not causing problems, such as loss of growth factors and nutrients, for example, blood plasma, contained in the fibrin-gel, and damage to platelet adhering to the fibrin-gel surface, the problems which have been pointed out in the conventional flattening by gauze.

Further, the compression device in the preferable aspect of the present invention employs the configuration in which a shape difference is provided between a shape of the convex part and a shape of the concave part or a stopper member is provided on at least one of the handle parts to provide a slight (for example, about 1 mm to 2 mm) separation distance (namely, a clearance) left between the convex part and the concave part when compressing the fibrin-gel (when bringing the gap between the pressing convex part and the accommodating concave part into a closed state), thereby making it possible to appropriately drain water (blood plasma component) seeping out of the fibrin-gel at compression, from the side area of the device.

Further, in the compression device in the preferable aspect of the present invention, a nonslip surface is formed at a portion of the accommodating concave part, the pressing convex part, or both of the members, thereby making it possible to appropriately grip the fibrin-gel when picking and compressing the fibrin-gel (nonslip effect).

In particular in the preferable aspect in which the above-described nonslip surface is formed of many pinholes, another advantageous effect, to make it possible to appropriately drain the water seeping out of the fibrin-gel through the pinholes, is achieved in addition to the nonslip effect.

Further, the compression device in the preferable aspect of the present invention has a simple configuration made by just combining two spoon-shaped members together for anyone to easily operate. Further, this configuration suppresses the production cost. Further, the configuration in which the pressing convex part and the accommodating concave part are joined to each other at other ends of the handle parts to be superposed one above the other while variably keeping the separation distance therebetween, significantly facilitates the operation of picking or compressing the fibrin-gel.

Further, in the compression device in the preferable aspect of the present invention, the first and second members are members containing at least one of paper, ABS resin (acrylonitrile butadiene styrene copolymer synthetic resin), PE (polyethylene), PS (styrol), PVC (vinyl chloride), MA (acrylic), PET (polyethylene terephthalate), PP (polypropylene), PMP (polymethylpentene), and PC (polycarbonate), so that the fibrin-gel compression device of the present invention is adaptable to disposable usage that is becoming the mainstream in the fields of dentistry medical treatment and the like.

Though the conventional gauze is usually discarded once it is used for flattening, in the compression device in the preferable aspect of the present invention, the first and second members are members containing at least one of stainless steel, titanium, aluminum, PP (polypropylene), PMP (polymethylpentene), and PC (polycarbonate) and can therefore be used any number of times by being subjected to cleaning or sterilization processing, and are also excellent in terms of environmental harmony.

Furthermore, according to the preferable aspect of the present invention, the configuration in which the fibrin-gel compression device has been previously subjected to sterilization processing is employed, thereby making it possible to further promote the efficiency of the work such as operation and the like.

### Brief Description of Drawings

[FIG. 1] A perspective view schematically illustrating a fibrin-gel compression device in Example 1.
[FIG. 2] A plan view of the fibrin-gel compression device in Example 1 illustrated in FIG. 2(a), a front view illustrated in FIG. 2(b), and a bottom view illustrated in FIG. 2(c).
[FIG. 3] Views explaining the usage state of the fibrin-gel compression device in Example 1, FIG. 3(a) illustrating a sectional view of the device in its opened state, and FIG. 3(b) illustrating a sectional view of the device in its compressed state.
[FIG. 4] A perspective view schematically illustrating a fibrin-gel compression device in Example 2.
[FIG. 5] FIG. 5(a) illustrating a plan view of the fibrin-gel compression device in Example 2, FIG. 5(b) illustrating a sectional view in a state that the device is opened, and FIG. 5(c) illustrating a sectional view in a state that the device is closed.
[FIG. 6] A view showing images of the fibrin-gel before and after compression.
[FIG. 7] Enlarged images (SEM images) showing the fibrin-gel compressed by the device of the present invention (compressed PRF).
[FIG. 8] SEM images showing the fibrin-gel compressed by usual gauze.
[FIG. 9] A view showing a model of full-thickness skin defects on the back of a mouse for verification experiment and temporal change in thickness of granulation tissue.
[FIG. 10] A diagram illustrating verification results showing the angioplasty activational effect of the compressed PRF.
[FIG. 11] Images showing an alveolar bone part at and after an operation of periodontal regenerative therapy using the compressed PRF.

### Best Mode for Carrying out the Invention

Hereinafter, the present invention will be described based on the following concrete embodiments referring to the accompanying drawings, but the present invention is not limited to any of the embodiments.

### (Example 1)

### (Fibrin-Gel)

First, a fibrin-gel 30 that is an object to be compressed by a fibrin-gel compression device 1 of the present invention will be briefly described. FIG. 6(a) shows the fibrin-gel 30 before compression. The fibrin-gel 30 is a gelatinous substance prepared using the centrifugal force by a centrifuge and an inherent coagulation system of blood plasma, as shown in the figure. The fibrin-gel 30 is prepared, in terms of formation state, only by adjustment of the number of rotations of centrifugation, and is therefore free of animal-derived factors, such as bovine thrombin and free of added calcium as well as has appropriate softness and formability to be also excellent in operability.

The fibrin-gel 30 has the above advantageous characteristics, so that the medical treatment using the fibrin-gel 30 is becoming the mainstream in fields of regenerative medicines for periodontal disease and the like and cosmetic surgeries. Note that, as one example of the centrifuge for converting the fibrin-gel 30 into a gelatinous form, a centrifuge having a trade name "MEDIFUGE" is commercially available from Corefront Corporation (see Non-Patent Document 2).

When implanting the fibrin-gel 30, an operator usually slices it or flattens it by gauze as described above. The flattening by gauze is pointed out as having disadvantages that the gauze also absorbs growth factors and nutrients (blood plasma components) contained in the fibrin-gel 30 and damages platelet adhering to the surface of the fibrin-gel as described above.

### (Outline of Fibrin-Gel Compression Device)

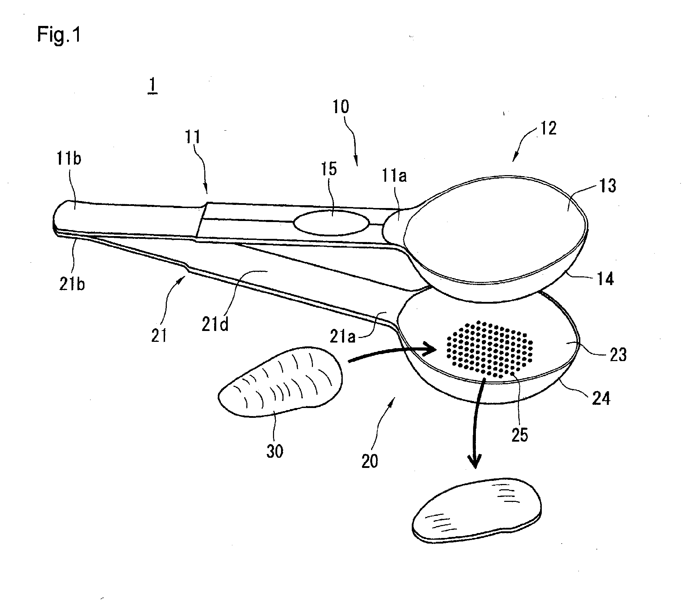

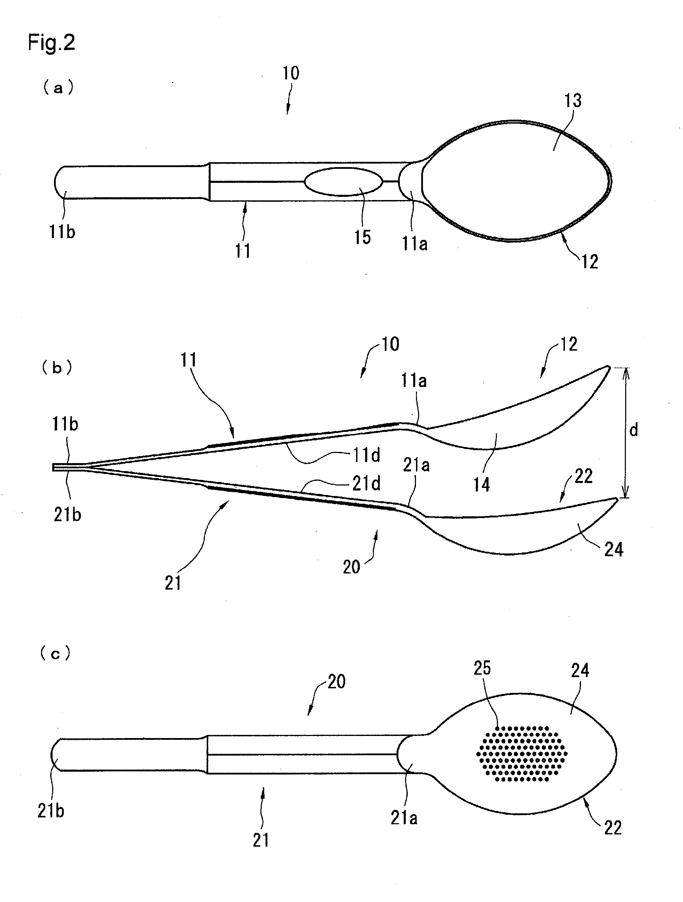

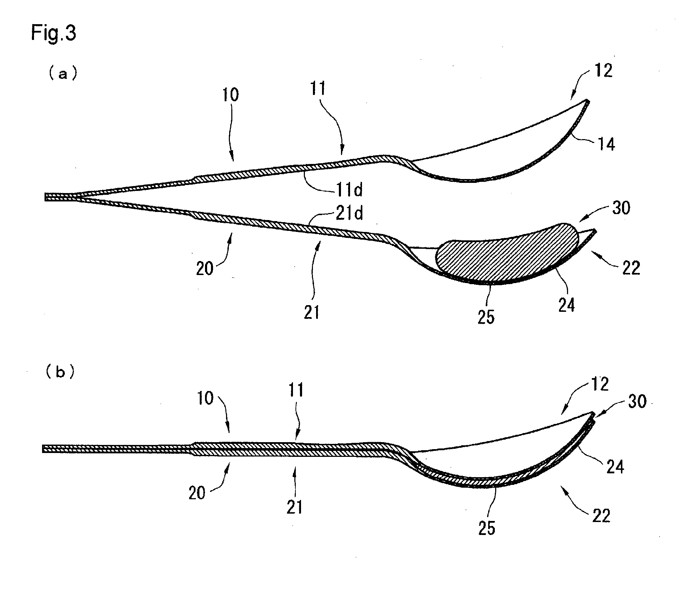

FIG. 1 is a perspective view schematically illustrating the fibrin-gel compression device 1 in Example 1. Further, FIG. 2(a) illustrates a plan view of the fibrin-gel compression device 1 in Example 1, FIG. 2(b) illustrate a front view, and FIG. 2(c) illustrates a bottom view. Further, FIG. 3 is views explaining the usage state of the fibrin-gel compression device in Example 1. More specifically, FIG. 3(a) illustrates a sectional view of the device in its opened state, and FIG. 3(b) illustrates a sectional view of the device in its compressed state.

Roughly speaking, the fibrin-gel compression device 1 of the present invention is composed of a first member 10 and a second member 20 (a first spoon-shaped member 10 and a second spoon-shaped member 20 in Example 1). The first spoon-shaped member 10 has a handle part 11 and a pressing convex part 12 coupled to one end 11a of the handle part 11. On the other hand, the second spoon-shaped member 20 similarly has a handle part 21 and an accommodating concave part 22 coupled to one end 21 a of the handle part 21.

Furthermore, the first and second spoon members 10, 20 are characterized by being joined to each other at other ends 11b, 21b of the handle parts 11, 21 so as to be superposed one above the other while variably keeping a separation distance d between the pressing convex part 12 and the accommodating concave part 22.

The portions of the handle parts 11, 21 joined as described above serve as a function similar to a grip part of usually commercially available tweezers. More specifically, the handle parts 11, 21 gradually separate from each other with the other ends 11b, 21b as base points, so that the convex parts 12 and the concave part 22 located at the tips of the handle parts 11, 21 have a predetermined separation distance d just as they are (in an open state without any force applied thereto) (FIG. 2(a)). Note that for appropriately placing the fibrin-gel 30, that is frequently used at the scene of medical treatment, on an inner surface 23 of the accommodating concave part 22, the separation distance d in the open state is preferably set to about 10 mm to 50 mm. Note that inside surfaces 11d, 21d where the handle parts 11, 21 face each other are preferably flat surfaces to make the separation distance d as little possible, in a state that the first and second spoon members 10, 20 are pushed to be superposed one above the other (in a closed state), making it possible to compress the fibrin-gel 30 into a more uniform small thickness.

The joint of the handle parts 11, 21 imparts an expansion and contraction property of the separation distance d attribute to elastic deformation of the handle parts 11, 21 using the joined point as a base point (in other words, similar to the elasticity of usual tweezers) to the fibrin-gel compression device 1, facilitating the picking, holding or compressing of the fibrin-gel 30 (see FIG. 2(b)). Note that the method of joining the handle parts 11, 21 is not particularly limited but is preferably welding, screwing, riveting or the like in terms of manufacture when the first and second spoon members 10, 20 are made of metal.

Note that the accommodating concave part 22 has a shape (curved shape) corresponding to the pressing convex part 12. This configuration ensures that when placing the fibrin-gel 30 on the inner surface 23 of the accommodating concave part 22 and compressing the fibrin-gel 30 using an outer surface 14 of the pressing convex part 12, the separation distance d between the outer surface 14 and the inner surface 23 becomes substantially uniform in terms of plane, making it possible to compress the fibrin-gel 30 into a uniform thickness (about 1 to 2 mm) (see FIG. 3(b)). To more effectively exhibit this function, it is more preferably that the handle part 21 of the second spoon-shaped member 20 also has a dimension almost the same as that of the handle part 11 of the first spoon-shaped member 10 (in particular, the length and the cross-sectional shape).

Incidentally, it is preferable to provide a slight shape difference between the curved shape of the outer surface 14 of the convex part 11 and the curved shape of the inner surface 23 of the concave part 22 or provide a stopper (not illustrated), such as a projection or the like at a portion of at least one of the handle parts 11, 21, so that a certain degree of the separation distance d (for example, about 1 mm to 2 mm) is left even if the first and second spoon members 10, 20 are brought into a state that they are completely pushed to be superposed one above the other. This configuration enables appropriate drainage of excessive water in the fibrin-gel 30 from a clearance between the convex part 11 and the concave part 22 at a later-described operation of compressing by the device 1 of the preset invention.

Further, both of the convex part 12 and the concave part 22 are each preferably formed in an ellipse or a circle in a plan view and curved to be recessed deepest at the center of the inner surface 13, 23 like a usual spoon used as tableware. This makes it possible to place the fibrin-gel 30 on the inner surface 23 of the accommodating concave part 22, and to keep the fibrin-gel 30 at the center of the inner surface 23 during compressing the fibrin-gel 30. Note that the dimension of the first and second spoon members 10, 20 is preferably slightly larger than that of the usual tableware spoon. For example, when both of the convex part 12 and the concave part 22 are formed into an ellipse, the length of the major axis is about 60 mm, the length of the handle parts 11, 12 is about 110 mm, and the whole length of the first and second spoon members 10, 20 is about 180 mm.

### (Nonslip Surface)

Further, it is preferable that a nonslip surface is formed at a portion of the accommodating concave part 22, the pressing convex part 12 or the accommodating concave part 22 and the pressing convex part 12 in the compression device 1 of the present invention. Examples of the nonslip surface include, for example, a surface with surface roughness greater than that of surfaces at other portions, a surface with a cross-sectional shape of continuous asperities or waves, a surface provided with many projections (not illustrated), a surface perforated with many pinholes 25 and so on. Note that the pinhole 25 may be a through hole (a hole penetrating from the inner surface 23 to the outer surface 24 of the accommodating concave part 22 in the illustrated example) or may be a non-through hole. The provision of the nonslip surface achieves advantageous effects such as appropriate gripping of the fibrin-gel 30 at picking and during compression (nonslip effect). Note that the nonslip surface is formed only in the one, that is, the accommodating concave part 22, in Examples 1 and 2 illustrated in the drawings.

### (Pinhole)

It is more preferable as the form of the above-described nonslip surface that many pinholes (through holes in the illustration) 25 are formed at a portion (the center portion in the illustration) of the accommodating concave part 22 as in the illustration. This allows a certain amount of water contained in the fibrin-gel 30 to be appropriately drained through the pinholes 25 provided in the accommodating concave part 22, namely, the certain amount of water to drip under its own weight when compressing the fibrin-gel 30, in addition to provision of the nonslip effect. Note that the pinholes 25 can be formed using the machining techniques such as drilling, electric discharge machining or the like.

### (Dimension of Pinhole)

The diameters φ of the pinholes 25 are more preferably within a range of 0.4 mm to 1.0 mm. A diameter φ less than 0.4 mm is not preferable because it is difficult to achieve the above-described nonslip effect and the drainage effect of water in the fibrin-gel (water drainage effect) and it is also difficult to remove dirt adhering to the pinholes 25 at cleaning of the device, as will be described hereinafter. On the other hand, a diameter φ exceeding 1.0 mm is not preferable because conspicuous marks are made on the compressed fibrin-gel 30, namely, it is difficult to flat the surface of the compressed fibrin-gel 30 after the compression.

### (Arrangement of Pinholes)

Note that when the pinholes 25 having the above-described preferable diameter φ are provided at the center of the concave part 22 forming an ellipse with a length of a major axis of about 60 mm in a plan view as illustrated in FIG. 2(c), 50 to 200 pinholes 25 in total will be formed. In the illustrated example, the pinholes 25 are arranged to form many lines, have a pitch of about 2 mm between the pinholes 25 adjacent to each other in each line, and be offset in a staggered arrangement with respect to the pinholes 25 in adjacent lines. The arrangement in terms of density of holes (the number of holes per unit area) preferably has 0.05 to 0.2 holes/mm². The shape and arrangement enables appropriate gripping and compression of the fibrin-gel 30 or drainage of water therein while suppressing a local increase in pressure.

Further, the handle part 11 of the first spoon member 10 is preferably provided with a thumb contact part 15. Putting the thumb of the operator on the thumb contact part 15 and placing the other fingers on the handle part 21 of the second spoon member 20 facilitates adjustment of the compression force and the separation distance d which are required when picking or compressing the fibrin-gel 30. In other words, the provision of the thumb contact part 15 improves the operability of the fibrin-gel compression device 1.

### (Example 2)

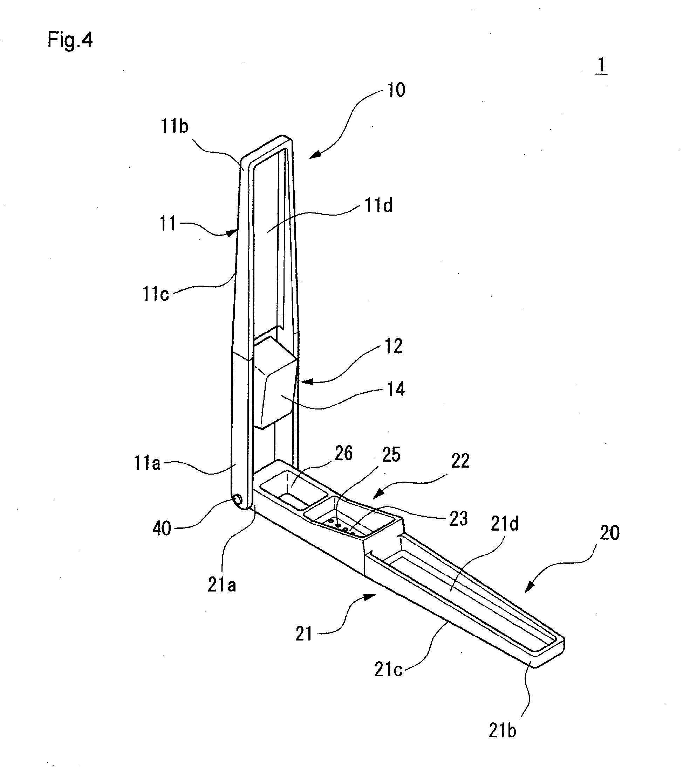

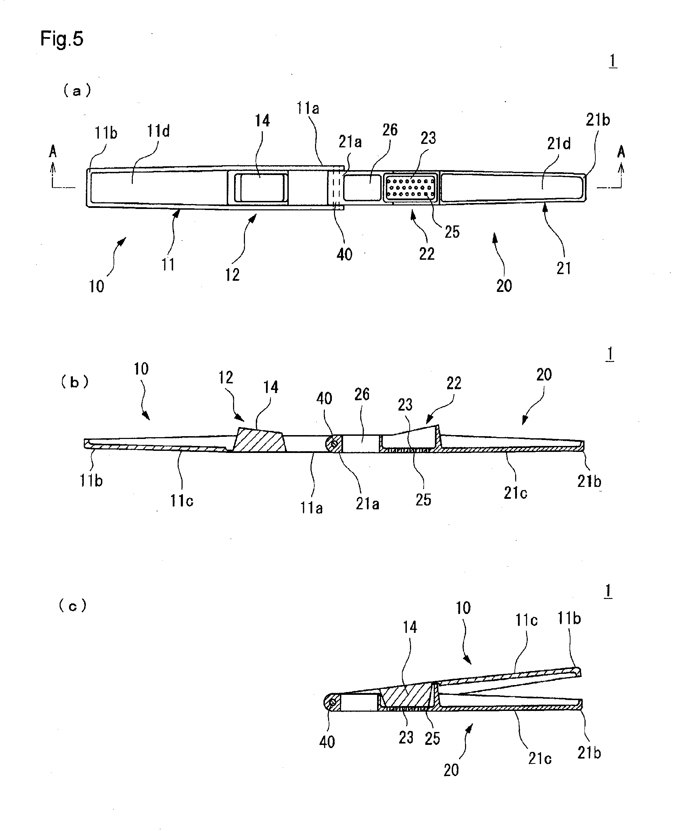

Next, a fibrin-gel compression device 1 according to another example (Example 2) of the present invention will be described referring to FIG. 4 an FIG. 5. FIG. 4 is a perspective view schematically illustrating the fibrin-gel compression device 1 in Example 2. FIG. 5(a) illustrates a plan view of the fibrin-gel compression device 1 in Example 2. FIG. 5(b) is a sectional view broken along a line A-A in FIG. 5(a) and illustrates a state that the device 1 is opened around a pin 40. FIG. 5(c) illustrates a sectional view in a state that the device 1 is closed around the pin 40. Note that the same numerals are used for components in the compression device 1 in Example 2 illustrated in FIG. 4 and FIG. 5 corresponding to the components in the device 1 in Example 1.

The fibrin-gel compression device 1 in Example 2 includes, similarly in Example 1, a first member 10 and a second member 20. The first member 10 has a handle part 11 and a pressing convex part 12 having an outer surface 14 that comes into contact with and presses the fibrin-gel 30. On the other hand, the second member 20 has a handle part 21 and an accommodating concave part 22 having an inner surface 23 that allows the fibrin-gel 30 to be placed thereon and accommodates the fibrin-gel 30.

However, in Example 2, one ends 11a, 21a of the handle parts 11, 22 extend farther from both sides of a pressing convex part 12 or the accommodating concave part 22 that hold and compress the fibrin-gel 30, and are rotatably supported to each other by a pin 40. Note that outside surfaces 11c, 21c of the handle parts 11, 21 are preferably flat as in the illustration, thereby enabling storage of the compression device 1 in a stable state even in unused time.

Further, the outer surface 14 of the pressing convex part 12, in a state that the outside surface 11c is located to the ground as illustrated in FIG. 5(b), is preferably gradually inclined to increase in thickness of the pressing convex part 12 in a direction away from the end 11a where the pin 40 is inserted. This makes it possible, at the time when compressing the fibrin-gel 30 accommodated in the accommodating concave part 22 as illustrated in FIG. 5(c), to keep the inner surface 23 of the accommodating concave part 22 and the outer surface 14 of the pressing convex part 12 substantially parallel with each other, thereby compressing the fibrin-gel 30 with the pressure made more uniform to obtain the fibrin-gel 30 having a uniform small thickness.

Further, the compression device 1 in Example 2 preferably has, similarly in Example 1, many pinholes 25 formed at the center of the bottom part of the accommodating concave part 22 because of the reason described in Example 1.

In addition, to reduce the raw material cost and reduce the weight of the device 1, inside surfaces 11d, 21d of the first and second members 10, 20 may have concave shapes in which the materials therein are eliminated and only outer peripheral walls are erected as illustrated in FIG. 4. Further, for the same reason, a bore 26 may be formed in the second member 20.

### (Cleaning and Reuse of Fibrin-Gel Compression Device)

The fibrin-gel compression device 1 of the present invention is implemented in a simple structure in which at least two members, associated with each other, are merely combined together as in the above-described Examples 1 and 2, so that the whole device can be easily cleaned and reused by applying autoclave sterilization or ultrasonic cleaning thereto. The first and second members 10, 20 are preferably made of a material that has pressure resistance, heat resistance, and corrosion resistance with respect to the cleaning, for example, at least one material among metals such as stainless steel, titanium, aluminum and the like, PP (polypropylene), PMP (polymethylpentene), and PC (polycarbonate).

### (Fibrin-Gel Compression Device responding to Disposable Usage)

On the other hand, the device 1 of the present invention can be manufactured not using the aforementioned material that has pressure resistance, heat resistance, and corrosion resistance. For example, in the case of using, for the material of the first and second members 10, 20, paper, plastic without the properties of the aforementioned heat resistance and so on (at least one of, for example, ABS resin (acrylonitrile butadiene styrene copolymer synthetic resin), PE (polyethylene), PS (styrol), PVC (vinyl chloride), MA (acrylic), and PET (polyethylene terephthalate)), the device 1 is single-use disposable. However, the disposable usage provides advantages in the efficiency of work, the safety in terms of infection prevention, the manufacturing cost and so on because many devices in the medical field are increasingly made disposable. Further, the resin materials with heat resistance and chemical resistance such as PP (polypropylene), PMP (polymethylpentene), and PC (polycarbonate) previously exemplified as the reusable resin are also often single-use disposable at the scene of the medical field, and therefore can be used even in a disposable specification.

Furthermore, the first and second members 10, 20 are preferably covered with a fluorocarbon resin or polymethylmethacrylate (PMMA). Note that examples of the fluorocarbon resin include PTFE (polytetrafluoroethylene), PFA (perfluoroalkoxy alkane), ETFE (ethylene-tetrafluoroethylen copolymer), FEP (perfluoroethylene-propne copolymer), ECTFE (ethylene-chlorotrifluoroethylene copolymer), polyvinylidene fluoride and so on. This provides an effect of making it difficult for blood and the like to adhere to the fibrin-gel compression device 1 in use.

Further, the device 1 of the present invention (any of the above-described reusable device and disposable device) may have been previously subjected to sterilization processing. The previous sterilization processing is preferable because it eliminates the necessity of cleaning and sterilization before (immediately before) use of the device 1 of the present invention and, in particular in disposable usage, significantly promotes the efficiency of work such as operation and so on.

Examples of the previous sterilization processing include ethylene oxide gas sterilization, gamma radiation sterilization, electron beam sterilization, hydrogen peroxide gas sterilization and so on. The gamma radiation sterilization is preferable from the viewpoint of easiness of sterilization processing, and the ethylene oxide gas sterilization and the electron beam sterilization are preferable from the viewpoint of less constraints of sterilizable material and shape. In particular, the ethylene oxide gas sterilization is most preferable in that it can sterilize almost all of materials. In the sterilization processing other than the ethylene oxide gas sterilization, poor conditions such as change of color, deterioration, adsorption of sterilizer may occur depending on selection of material.

### (Example 3)

### (Method of Compressing Fibrin-Gel using Fibrin-Gel Compression Device)

Next, a method of using the fibrin-gel compression device of the present invention will be exemplified. Here, it is assumed that the fibrin-gel compression device 1 described in Example 1 is used.
(1) First, a vacuum blood collection tube containing collected blood (about 10 ml) is directly placed inside a centrifuge for preparation of fibrin-gel (for example, MEDIFUGE, distributor: Corefront Corporation, manufacturer: Silfradent s.r.l., ITALY) and subjected to centrifugation processing for a predetermined period.
(2) After the centrifugation processing, the gelled fibrin-gel 30 is picked with tweezers, or the like, from the vacuum blood collection tube and placed on a plastic petri dish.
(3) Thereafter, an aggregated erythrocyte fraction adhering to one end of the fibrin-gel is cut with scissors or the like.
(4) At this point in time, the fibrin-gel 30 still contains a sufficient amount of blood plasma component and takes a shape of a bullet or a cod roe (see FIG. 6(a)).
(5) The fibrin-gel 30 is compressed by the fibrin-gel compression device 1. More specifically, the fibrin-gel is placed on the accommodating concave part 22 of the device, and then the fibrin-gel 30 is pressed with the pressing convex part 12 for about 10 seconds. Note that excessive blood plasma component (water) drains through the pinholes 25 under its own weight but the drainage amount is sometimes not sufficient, and therefore the device 1 is preferably slightly tilted to drain water through the clearance between the convex part 12 and the concave part 22 to thereby drain the blood plasma component.

### (State of Fibrin-Gel after Compression)

The above compression operation was actually carried out using the device 1 in Example 1. As a result, the weight of the fibrin-gel 30 before the compression was about 1.5 g, whereas the weight after the compression was about 0.3 g. This means that the blood plasma component (water) of about 1.2 g was drained from the fibrin-gel 30.

FIG. 6(b) is an image showing the whole fibrin-gel after the compression. As shown in FIG. 6(b), the fibrin-gel 30 was divided into three sections (named A, B, and C respectively), and the surface states of the sections A. B, C were photographed with a scanning electron microscope (SEM). Circles with broken lines in the drawing show sampling positions photographed with SEM.

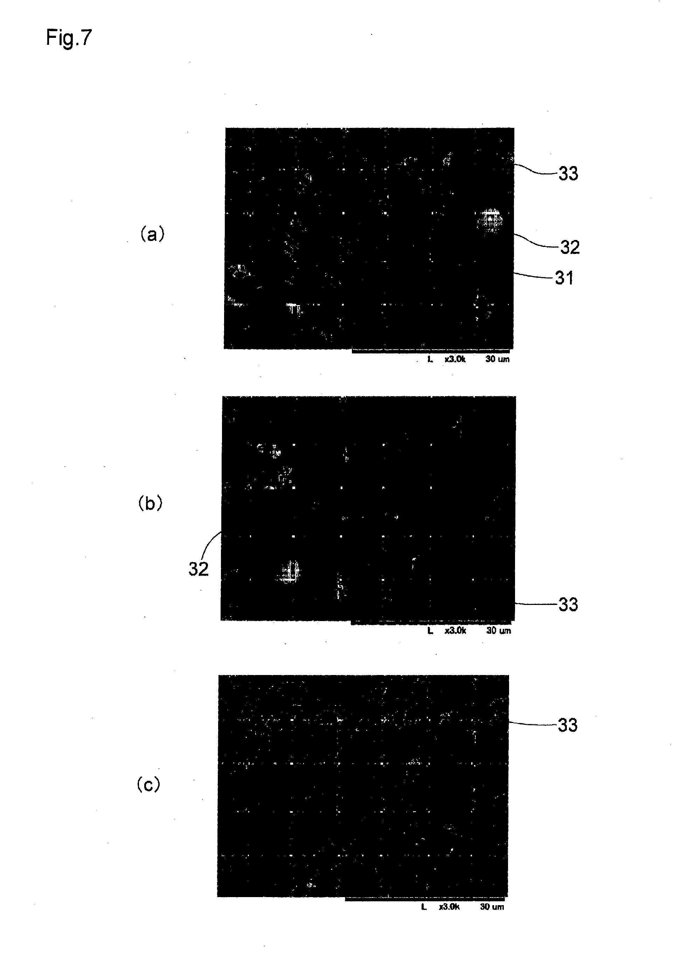

FIGs. 7(a), (b), (c) are enlarged images showing the surface states at the sampling positions of the sections A, B, C shown in FIG. 6(b). Many small spherical bodies 31 observed in the images in FIGs. 7(a) and (b) are platelets and spherical bodies 32 larger than the platelets are lymphocytes. In addition, existence of mesh-like structure 33 is observed everywhere in the drawings. The mesh-like structure 33 is very important because when the fibrin-gel 30 is implanted into a living organism and cells invade thereinto, the mesh-like structure 33 serves as a scaffold for the cells to stay there so as to exhibit a function to promote tissue regeneration.

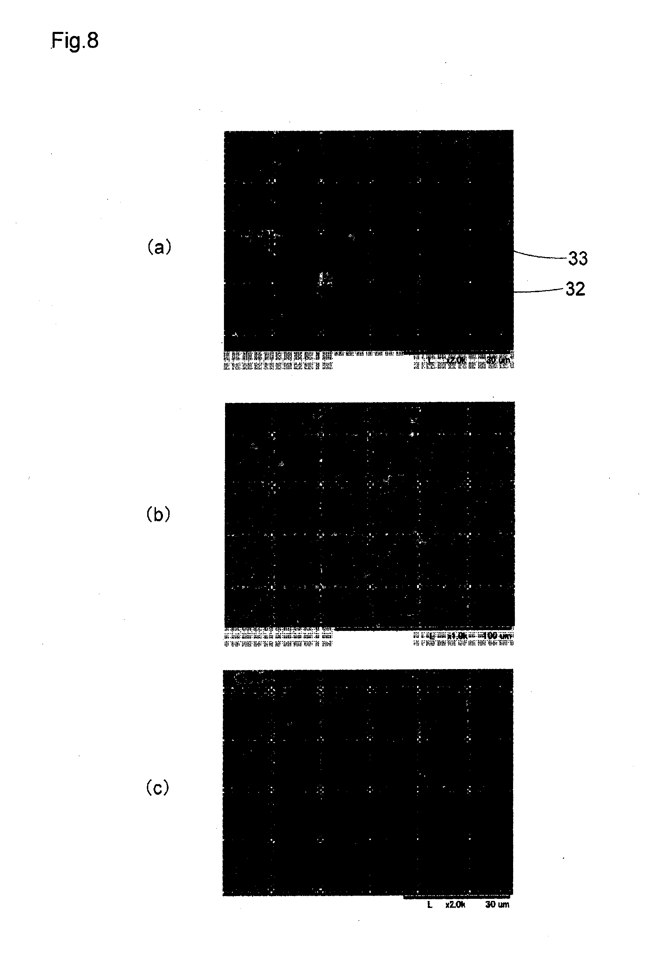

Note that as a comparative experiment, fibrin-gel with a similar size was compressed by usual gauze. FIGs. 8(a), (b), (c) show SEM images at the same positions as the sampling positions shown in FIG. 6(b). In these drawings, the observed platelets 31 and lymphocytes 32 were extremely small in number as compared to those in the case of FIG. 7 and their shapes were deformed. Further, almost all the above-described mesh-like structure 33 was lost. A small amount of remaining mesh-like structure 33 can be observed, which means that the compression by gauze is not uniform. In addition, the mesh-like structure 33 looks like as if it was flattened more as compared to the case in FIG. 7. As described above, the fibrin-gel 30 compressed by gauze cannot build the scaffold for the cells to stay in the living organism and is therefore considered to be unable to sufficiently exhibit the function to promote tissue regeneration and accordingly a wound-healing effect.

### (Example 4)

### (Verification of Wound-Healing Effect: Model of Full-thickness Skin Defects on Back of Mouse)

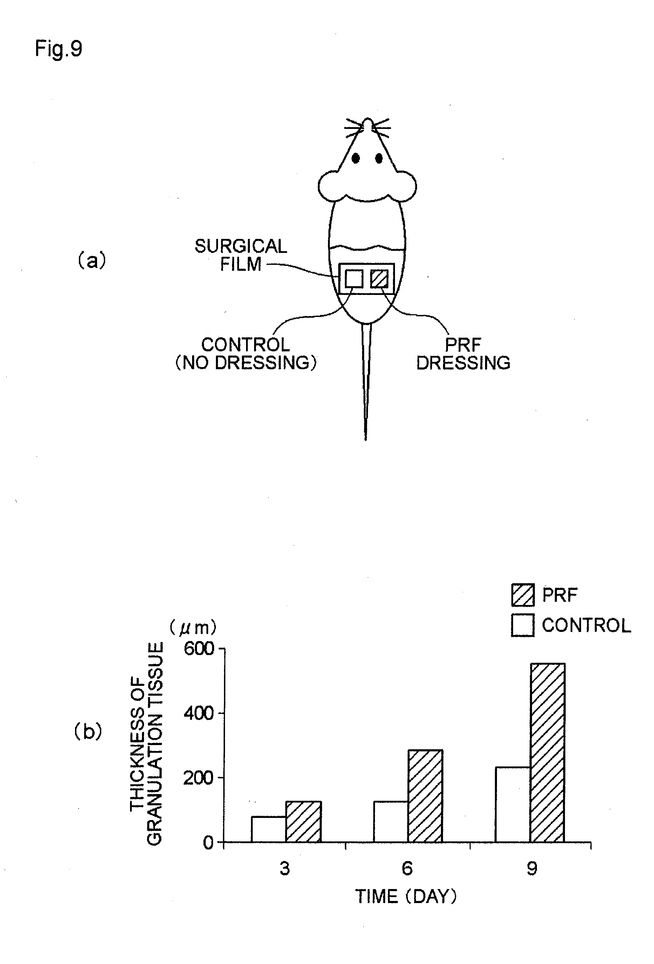

To verify the wound-healing effect by the fibrin-gel 30 compressed by the device 1 of the present invention (hereinafter, simply referred also to as compressed PRF or PRF), an animal experiment using a mouse was carried out. Specifically, as illustrated in FIG. 9(a), the full-thickness of skin on the back of an ICR mouse (5-week-old male) was made defective to prepare two adjacent defective regions (regions with a size of 7 mm × 7 mm). One of the defective regions was dressed with fibrin-gel with a size of 10 mm × 10 mm (PRF in the drawing). The other defective region was not dressed with PRF (called CONTROL in this experiment). In a manner to cover the regions, a surgical film (trade name: Tegaderm, manufacturer: 3M) was applied to the back of the mouse.

After observation of the sectional images (not shown) of the two defective regions for several days, it was found that PCNA (Proliferating cell nuclear antigen) positive cells significantly increased in the defective region dressed with PRF (namely, a peripheral portion of PRF) as compared with CONTROL (without being dressed with PRF). It was also observed that PCNA positive cells increased not only at the peripheral portion of PRF but also inside PRF (PRF itself). Further, FIG. 9(b) illustrates temporal change in thickness of granulation tissue at the defective regions. The thickness of the granulation tissue here shows the degree of tissue regeneration. As illustrated in FIG. 9(b), it was found that the thickness of the granulation tissue at the defective region dressed with PRF more significantly increased with time as compared with the case of CONTROL. From the above-described observation results of the PCNA positive cells and the thickness of the granulation tissue, it can be said that PRF compressed by the device 1 of the present invention exhibits an excellent wound-healing effect with respect to a mouse.

### (Example 5)

### (Verification of Vascularization Effect: CAM assay)

To verify the vascularization effect by the compressed PRF provided by the present invention, CAM assay (chick chorioallantoic membrane assay) was used. In CAM without administration of PRF (called CONTROL in this experiment), maturation of preexisting blood vessels and formation of new microvessels were verified in CAM on the fourteenth day after egg laying, and the fact that blood vessels ran uniformly and in a mesh form in the whole CAM was observed.

On the other hand, in the case where PRF was administered to CAM on the eleventh day after egg laying, formation of many new microvessels was verified in CAM after a lapse of three days after the administration (namely, on the fourteenth day after the egg laying). In addition, it was observed that preexisting blood vessels were pulled toward PRF and, as a result, blood vessels ran radially outward from PRF.

Further, PRF subjected to glutaraldehyde fixation (also called GA processing) (namely, a model in which growth factors contained in PRF were deactivated) was prepared and administered to CAM on the eleventh day after the egg laying. In the case of administration of the GA-processed PRF, the above-described movement of the preexisting blood vessels and formation of new microvessels were not verified even in CAM after a lapse of three days after the administration (namely, on the fourteenth day after the egg laying), but the run of blood vessels similar to that in the case of CONTROL was observed.

The following Table 1 shows the average numbers of blood vessels on CAM (CAM images) observed on the fourteenth day after the egg laying. A remarkably larger number (with a significant difference) of blood vessels were observed in the case of administration of PRF than in the case of CONTROL and in the case of administration of PRF (GA processed). From this result, it can be said that PRF compressed by the device of the present invention provides the effect to promote vascularization.

**(Table 1)**

| | CONTROL | PRF (GA) | PRF |
|---|---|---|---|
| AVERAGE NUMBERS OF BLOOD VESSELS | 34.0±12.7 | 39.3±8.0 | 59.2±8.6 |

### (Example 6)

### (Verification of Angioplasty Activational Effect: Human Vessel Endothelial Cell)

A plurality of human vessel endothelial cells in which scratches (portions where cells peeled off) with a predetermined width were formed were prepared. Specifically, these human vessel endothelial cells were immersed in culture media filled in test cells (6-well Cell Culture Insert Companion Plate with lid, manufacturer: Becton, Dickinson and Company). The compressed PRF was immersed near the endothelial cell via a membrane having microscopic pores (Cell Culture Inserts, manufacturer: Becton, Dickinson and Company) in the culture media of one of the cells, whereas the compressed PRF was not immersed in the culture media of the other of the cells (this condition being called CONTROL in this experiment), and the temporal changes in widths of the scratches (clearances) in the endothelial cells in both the culture media were observed.

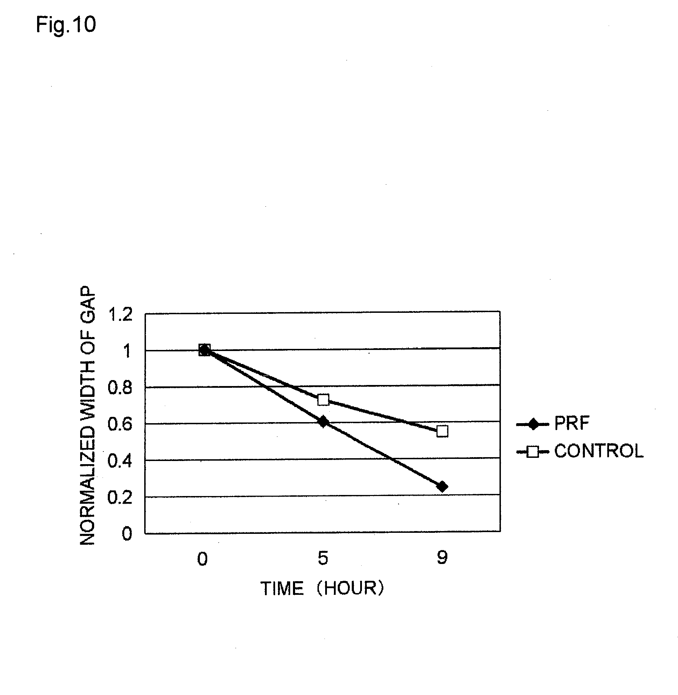

FIG. 10 illustrates the observation results. The horizontal axis indicates the elapsed time and the vertical axis indicates the normalized scratch width (namely, a value obtained by dividing each measured value by an initial width (at formation of the scratch)). It is found that the scratch width of the cell, near which the compressed PRF provided by the present invention existed, becomes smaller in a shorter time as compared to CONTROL. In other words, immersion of the compressed PRF can be said to promote extension of the vessel endothelial cells. Accordingly, the compression PRF is also expected to provide an excellent effect to form blood vessels.

### (Example 7)

### (Experimental Proof by Periodontal Regenerative Therapy)

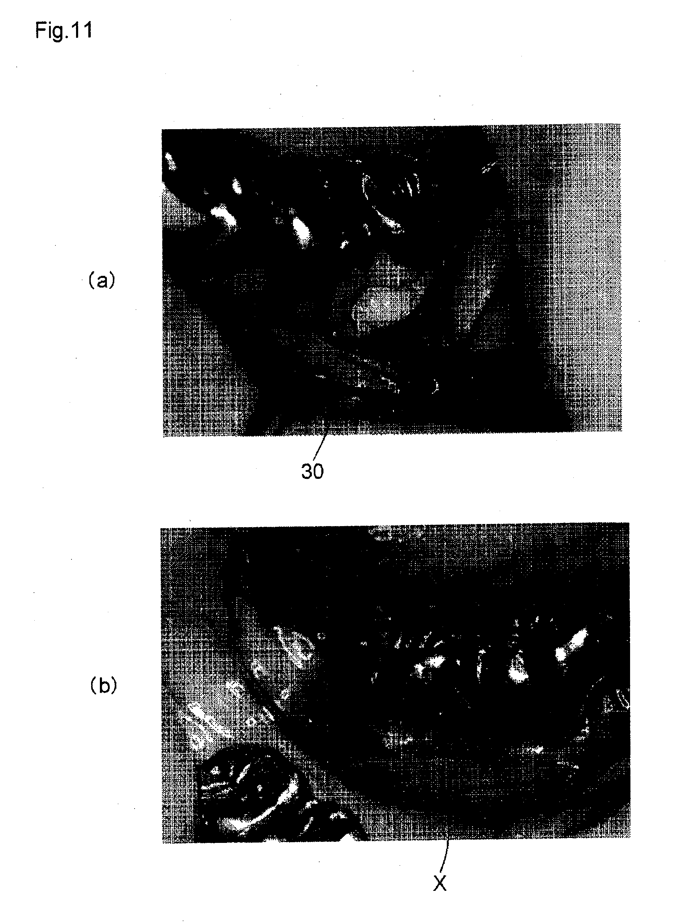

The compressed PRF provided by the present invention was applied to actual periodontal regenerative therapy, and the progress at and after the operation was observed. In the operation, an alveolar bone defective part of a patient was kept clean, and the defective part was filled with ceramic and then covered with the compressed PRF (see a numeral 30 in FIG. 11(a)). The covered part is further covered with gingiva and sutured. In verification of the sutured part after one week and after one month of the operation, the wound never opened. In addition, the implanted ceramic was not expelled as a foreign substance. Note that FIG. 11(b) is a mirror image showing the state after one month, and a letter X indicates the sutured part. These results suggest that the compressed PRF has at least an effect to promote healing of a wound in soft tissue.

The fibrin-gel compression device 1 of the present invention has been described in details based on the above-described examples but is not necessarily limited to them. For example, the fibrin-gel compression device 1 in Example 1 is configured by combining two spoon-shaped members, but may be configured by combining more (for example, four) spoon-shaped members. Further, to increase the strength and stiffness of the device 1, a composite material containing glass fiber and carbon fiber (as one example, PET (polyethylene terephthalate) with glass fiber) may be used as the raw material of the first member and the second member.

As described above, the fibrin-gel compression device 1 of the present invention is a dedicated tool for compressing the fibrin-gel 30 created based on a very unique idea, and the dedicated tool as in the present invention has not been used or proposed in the field of regenerative medicines and the like as far as the inventors and the applicants know.

### (Industrial Applicability)

As described above, the medical treatment using the fibrin-gel is becoming the mainstream in fields of regenerative medicines for periodontal disease and the like and cosmetic surgeries, but there is no dedicated tool for compressing the fibrin-gel with excellent operability. Accordingly, the fibrin-gel compression device of the present invention contributes to development of secure and safe medical treatment and is very high in industrial applicability.

### Explanation of Codes

1 fibrin-gel compression device
10 first member (first spoon-shaped member)
11 handle part
11a, 11b end of handle part (one end, other end)
11c, 11d outside surface of handle part, inside surface of handle part
12 pressing convex part
13 inner surface of pressing convex part
14 outer surface of pressing convex part
15 thumb contact part
20 second member (second spoon-shaped member)
21 handle part
21a, 21b end of handle part (one end, other end)
21c, 21d outside surface of handle part, inside surface of handle part
22 accommodating concave part
23 inner surface of accommodating concave part
24 outer surface of accommodating concave part
25 pinhole
26 bore
30 fibrin-gel
31 comparatively small spherical body (platelet)
32 comparatively large spherical body (lymphocyte)
33 mesh-like structure
40 pin

## Claims

1. A fibrin-gel compression device **characterized by** comprising:
a first member having a handle part and a pressing convex part; and
a second member having a handle part and an accommodating concave part forming a shape corresponding to the pressing convex part,
wherein said first and second members are partially joined or rotatably supported to each other to variably adjust a separation distance between the pressing convex part and the accommodating concave part to be able to accommodate and compress fibrin-gel therebetween.

2. The fibrin-gel compression device according to claim 1,
wherein a shape difference is provided between a shape of the pressing convex part and a shape of the accommodating concave part or a stopper member is provided on at least one of the handle parts, to provide the separation distance of 1 mm to 2 mm when compressing the fibrin-gel.

3. The fibrin-gel compression device according to claim 1 or 2,
wherein a nonslip surface is formed at a portion of the accommodating concave part, the pressing convex part, or the accommodating concave part and the pressing convex part.

4. The fibrin-gel compression device according to claim 3,
wherein the nonslip surface is formed of many pinholes.

5. The fibrin-gel compression device according to claim 4,
wherein the pinholes are through holes.

6. The fibrin-gel compression device according to claim 4 or 5,
wherein the pinholes have a diameter of 0.4 mm to 1.0 mm and are provided at a hole density of 0.05 to 0.2 holes/mm².

7. The fibrin-gel compression device according to any one of claims 1 to 6,
wherein the pressing convex part and the accommodating concave part are formed in a spoon shape.

8. The fibrin gel compression device according to any one of claims 1 to 7,
wherein the pressing convex part and the accommodating concave part are joined to each other at other ends of the handle parts to be superposed one above the other while variably keeping the separation distance therebetween.

9. The fibrin-gel compression device according to any one of claims 1 to 8,
wherein said first and second members are members containing at least one of paper, ABS resin (acrylonitrile butadiene styrene copolymer synthetic resin), PE (polyethylene), PS (styrol), PVC (vinyl chloride), MA (acrylic), PET (polyethylene terephthalate), PP (polypropylene), PMP (polymethylpentene), and PC (polycarbonate), and said fibrin-gel compression device is disposable.

10. The fibrin-gel compression device according to any one of claims 1 to 8,
wherein said first and second members are members containing at least one of stainless steel, titanium, aluminum, PP (polypropylene), PMP (polymethylpentene), and PC (polycarbonate), and said fibrin-gel compression device is able to be subjected to sterilization processing or ultrasonic cleaning.

11. The fibrin-gel compression device according to any one of claims 1 to 10,
wherein said fibrin-gel compression device has been previously subjected to sterilization processing.
